# EUROPEAN PATENT SPECIFICATION

(11) **EP 2 643 461 B1**
(45) Date of publication and mention of the grant of the patent: **15.06.2016**
(21) Application number: 12829723.1
(22) Date of filing: 01.08.2012
(51) Int. Cl.: C12N 15/11, C12Q 1/70

(54) **METHODS, COMPOSITIONS, AND KITS FOR DETERMINING HEPATITIS A VIRUS**
VERFAHREN, ZUSAMMENSETZUNGEN UND KITS ZUM NACHWEIS DES HEPATITIS A-VIRUS
PROCÉDÉS, COMPOSITIONS ET TROUSSES DE DÉTECTION DU VIRUS DE L'HÉPATITE A

(30) Priority: 07.09.2011 US 201161531818 P
(43) Date of publication of application: 02.10.2013
(73) Proprietor: Grifols Therapeutics Inc., Research Triangle Park, NC 27709 (US)
(72) Inventor: BURDE, Stefan, Cary, NC 27519 (US); BUNO, Brett, Durham, NC 27707 (US); WRONSKA, Danuta, Raleigh, NC 27613 (US)
(74) Representative: Durán-Corretjer, S.L.P.
(86) International application number: PCT/US2012/049099
(87) International publication number: WO 2013/036332

(56) References cited:
- WO-A2-03/030624
- WO-A2-03/106641
- DE-B3-102006 034 844
- US-A- 5 702 891
- US-A1- 2003 124 517
- US-A1- 2004 072 150
- US-A1- 2006 014 142
- US-A1- 2006 014 142
- DATABASE GENBANK 09 September 2010 'Hepatitis A virus isolate G1S1-IRES 5' UTR', XP003031212 Database accession no. FJ829479.1
- KANDA, T. ET AL.: 'Analysis of 5' nontranslated region of hepatitis A viral RNA genotype I from South Korea: comparison with disease severities, art. e15139' PLOS ONE vol. 5, no. 12, 28 December 2010, pages 1 - 6, XP055066730

### SEQUENCE LISTING

<110> Grifols Therapeutics Inc. Burde, Stefan Buno, Brett Wronska, Danuta
<120> METHODS, COMPOSITIONS, AND KITS FOR DETERMINING HEPATITIS A VIRUS
<130> T126 2200WO
<150> US 61/531,818
   <151> 2011-09-07
<160> 3
<170> PatentIn version 3.5
<210> 1
   <211> 23
   <212> DNA
   <213> Artificial Sequence
<220>
   <223> Hepatitis A Virus
<400> 1
   gcgcccggcg gggtcaactc cat 23
<210> 2
   <211> 21
   <212> DNA
   <213> Artificial Sequence
<220>
   <223> Hepatitis A Virus
<400> 2
   agccaagtta acactgcaag g 21
<210> 3
   <211> 32
   <212> DNA
   <213> Artificial Sequence
<220>
   <223> Hepatitis A Virus
<400> 3
   ttagcatgga gctgtaggag tctaaattgg gg 32

## Description

### FIELD OF THE INVENTION

The present invention relates to Hepatitis A Virus (HAV) and includes methods, compositions, and kits for detecting same.

### BACKGROUND OF THE INVENTION

HAV is a RNA virus that causes a non-chronic infection of the liver and can be transmitted via blood products. Plasma donations collected for manufacture of biological theraputics undergo testing for HAV RNA and donations that test positive are rejected.

International Patent application number WO03/106641A2 discloses a method for the detection of Hepatitis A Virus (HAV) in a biological sample, e.g. blood, plasma, serum and blood cells, based on nucleic acid amplification techniques, which include PCR, transcription-mediated amplification or 5' nuclease assays as, for example, the TaqMan technique. Said diagnostic method is based on the use of a pair of primers with sequences: 5'-GGATTGATTGTCAGGGCTGTC-3' and 5'-CCCTCTCACAGGATCCCATTT-3'. It contemplates several amplification and detection strategies.

In addition, German Patent number DE102006034844B3 discloses a method for the detection of HAV and/or Parbovirus B19 in whole blood, plasma, serum or cellular blood components from individuals or pools of up to 96 individuals based on the performance of the TaqMan technique. For the detection of HAV it teaches the use of two specific primers with sequences: 5'-GGTAGGCTACGGGTGAAACCTCTT-3' and 5'-GTCAGTCCTCCGGCGTTGAATGG-3'; and two specific probes with sequences: 5'-TATGAAGAGATGCCTTGGATAGGGT-3' and 5'-TATGAAGAGATGCTTTGGATAGGGT-3'. Said probes have the same dye at 5' and at 3' the same dye or quencher. In addition, said document describes the use of an internal control to verify that the PCR has been correctly performed. Said internal control is added preferably to the lysis buffer.

There is still a need, therefore, for alternative compositions and methods for detecting HAV.

### SUMMARY OF THE INVENTION

There is now provided, in one aspect, an isolated nucleic acid molecule consisting of a nucleotide sequence, or a complement thereof, as set forth in:
5'-GCG CCC GGC GGG GTC AAC TCC AT-3' (SEQ ID NO:1);
5'-AGC CAA GTT AAC ACT GCA AGG-3' (SEQ ID NO:2); or
5'-TTA GCA TGG AGC TGT AGG AGT CTA AAT TGG GG-3' (SEQ ID NO:3).

In another aspect, the present invention provides a composition comprising a pair of oligonucleotide primers comprising a forward primer having a sequence as set forth in SEQ ID NO:1 and a reverse primer having a sequence as set forth in SEQ ID NO:2, wherein the pair of primers are capable of annealing to a target sequence under a PCR condition to amplify the target sequence.

In another aspect, the present invention provides a method for amplifying a target sequence, the method comprising:
performing a PCR with the target sequence as template, wherein performing comprises providing the PCR with a forward primer comprising the sequence as set forth in SEQ ID NO:1 and a reverse primer comprising the sequence as set forth in SEQ ID NO:2.

In one aspect, the present invention provides a method for determining HAV in a sample, the method comprising:
performing a PCR with a nucleic acid template in the sample using a forward primer comprising the sequence as set forth in SEQ ID NO:1 and a reverse primer comprising the sequence as set forth in SEQ ID NO:2; and
detecting an amplicon generated by the forward and the reverse primer, wherein the presence of the amplicon determines the HAV in the sample.

In still further aspects, the present invention provides a kit comprising the compositions and/or the one or more of the nucleic acid molecules of the present invention.

### DETAILED DESCRIPTION

There is now provided, in one aspect, an isolated nucleic acid molecule consisting of a nucleotide sequence as set forth in SEQ ID NO: 1, SEQ ID NO:2, or SEQ ID NO:3, or a complement thereof.

Also described is an isolated nucleic acid molecule comprising a nucleotide sequence as set forth in SEQ ID NO: 1, SEQ ID NO:2, or SEQ ID NO:3, or a fragment thereof having at least 10 nucleotides, illustratively, 10, 11, 12, 13, 14, 15, 16, 17, 18, 19, 20, 21, 22, 23, 24, 25, 26, 27, 27, 29, 30, 31, or 32 nucleotides.

In other embodiments, the isolated nucleic acid molecules of the present invention have a length of no more than about 100 base pairs, illustratively, no more than about: 100, 90, 80, 70, 60, 55, 50, 45, 40, 35, 34, 33, 32, 31, 30, 29, 28, 27, 26, 25, 24, 23, 22, 21, 20, 19, 18, 17, 16, 15, 14, 13, 12, 11, and 10 bps.

In another embodiment, the present invention provides an isolated nucleic acid molecule consisting of a nucleotide sequence selected from the group consisting of: SEQ ID NO: 1, SEQ ID NO:2, or SEQ ID NO:3, or a complement thereof.

The term "nucleic acid molecule" herein includes polymers composed of naturally-occurring nucleotide bases, sugars and covalent internucleoside (backbone) linkages as well as nucleic acid molecules having non-naturally-occurring portions that function similarly. Further, the term "nucleic acid molecule" also includes polymers that are double-stranded, single-stranded, comprising RNA, DNA, modified RNA or DNA, RNA or DNA mimetics, or any combination thereof.

In some embodiments, oligonucleotide primers and probes can be derived from the nucleic acid sequences disclosed herein. In various embodiments, primers and probes are used in combination with each other. The present invention finds use in a variety of different applications including, but not limited to, research, medical, and diagnostic applications for HAV. For example, primers and probes can provide for reagents for use in, for example, an HAV detection assay and/or kit.

Generally, a pair of primers comprising a forward primer and a reverse primer can provide for specific amplification (e.g., by PCR) of a target nucleic acid flanked by the primers to produce an amplification product (also referred to as an "amplicon"). In this regard, each primer binds to its complementary or substantially complementary target sequence thereby providing a place for a polymerase to bind and extend each primer's 3' end by the addition of nucleotides thereby providing a complementary copy of the target sequence.

In one embodiment, a primer pair comprises a forward primer having the sequence as set forth in SEQ ID NO:1 and a reverse primer having the sequence as set forth in SEQ ID NO:2.

In one embodiment, the target nucleic acid is at least a segment of a cDNA prepared from reverse transcribed RNA of a HAV. One of ordinary skill in the art will recognize that RNA can be reverse transcribed using methods (e.g., reverse transcription (RT)) known in the art to provide a template for amplification by primers.

For example, in some embodiments, a reverse transcription-PCR (RT-PCR) is performed using a reverse transcriptase, a polymerase, and a pair of HAV-specific primers of this invention to amply HAV viral RNA in a sample. The sample can be a culture supernatant or a plasma specimen prepared from a HAV infected individual. The pair of HAV-specific primers of this invention is capable of amplifying HAV viral RNA.

Generally, a probe is an oligonucleotide that is complementary or substantially complementary to a nucleotide sequence of the target nucleic acid. Probes are useful for a variety of applications including, but not limited to, detecting or capturing the target nucleic acid or an amplicon corresponding to the target. For example, probes suitable for use in amplification-based detection methods can be designed from any sequence positioned within and/or comprising the sequence of an amplification product that would be produced using two selected primers.

In one embodiment, the probe comprises a nucleotide sequence complementary to or substantially complementary to at least a portion of a sequence of an amplicon generated by a primer pair comprising a forward primer having the sequence as set forth in SEQ ID NO:1 and a reverse primer having the sequence as set forth in SEQ ID NO:2.

In other embodiments, the probe comprises the nucleotide sequence as set forth in SEQ ID NO:3, or a complement thereof.

One skilled in the art will recognize that the isolated nucleic acid molecules of the present invention including primers and/or probes can be obtained by standard molecular biology techniques described in Current Protocols in Molecular Biology (1999. Ausubel FM, Brent R, Kingston RE, Moore DD, Seidman JG, Smith JA, Struhl K, editors. John Wiley & Sons, Inc.) or by chemical synthesis or by nucleic acid analogs. Methods involving chemical synthesis may be automated and commercially available and can include, for example, phosphodiester, phosphotriester, or phosphoramidite methods. U.S. Patent Nos. 4,458,066; 4,415,732; and Meth. Enzymol. 1979 68:90 and 109, disclose examples of chemical synthesis methods. Chemical nucleic acid synthesis allows for the incorporation of unnatural or modified bases, as well as a variety of labeling moieties, into a nucleic acid molecule. Further, modified backbone chemistries such as, for example, peptide linkages, phosphorothioates, phosphoroamidates, phosphotriesters, 2'-O-Methyl RNA, 2'-O-Mt RNA, P-Ethoxy DNA, and P-Ethoxy 2'-O-Mt RNA are also readily available and known in the art. Furthermore, the uses of cross-linkable probes in nucleic acid hybridization assays to cross-link to target sequences are known in the art. For example, compounds based on furocoumarin or psoralen attached to nucleic acid molecules through adduct formation are described in U.S. Pat. No. 4,826,967 and U.S. Pat. No. 5,082,934, describes a photoactivatible nucleoside analogue comprising a coumarin moiety linked through its phenyl ring to the 1-position of a ribose or deoxyribose sugar moiety in the absence of an intervening base moiety.

Nucleic acid analogs and mimics have similar chemical structures as native nucleic acid molecules but with unique modifications. Nucleic acid analogs, such as locked nucleic acids (LNAs), peptide nucleic acids (PNAs), and morpholinos, improve the capabilities of traditional nucleic acid molecules beyond the limitations associated with standard nucleic acids chemistry (Karkare S and Bhatnagar D. Appl. Microbial. Biotechnol. 2006 71:575-586.) Such nucleic acid analogs greatly expand and improve the capabilities to detect and identify related nucleic acid sequences.

In some aspects, an isolated nucleic acid molecule of the present invention further comprises one or more heterologous nucleotides. The term "heterologous nucleotides" herein refers to a nucleotide or nucleotides that are not a natural part of the isolated nucleic acid molecule but which are naturally or artificially joined to the isolated nucleic acid molecule. Examples of a heterologous nucleic acid sequence include, but is not limited to, a vector sequence, a sequence that is complementary to a base sequence of a purification probe, and a sequence comprising one or more restriction enzyme sites.

In one embodiment, the one or more heterologous nucleotides comprise a sequence that is complementary to a base sequence of a purification probe. The purification probe can be joined to solid supports such as, for example, a matrix or particles free in solution. Non-limiting examples of a solid support include nitrocellulose, nylon, glass, polyacrylate, mixed polymers, polystyrene, silane polypropylene, and magnetically-attractable particles. For example, the purification probe, which may comprise a DNA or RNA sequence, can be labeled with amine or biotin tags via a cross-linker. These biotin or amine labeled purification probes are then amenable to immobilization and detection strategies that allow *in vitro* nucleic acid:nucleic acid or protein:nucleic acid interactions. Thus, annealing of the heterologous segment of the isolated nucleic acid molecule with its complementary base sequence of the purification probe can facilitate sample purification of molecules that anneal virus-specific sequence segment of the isolated nucleic acid molecule. U.S. Patent No. 6,534,273, describes a method for capturing a target nucleic acid molecule in a sample onto a solid support.

In one embodiment, the isolated nucleic acid molecules of the present invention are joined to a solid support such as those described above.

In some embodiments, the one or more heterologous nucleotides comprise one or more repeating base sequences, for example, one or more repeating base sequences that are complementary to one or more repeating base sequences of the purification probe. A repeating base sequences can be a regularly repeating base sequence, such as those formed, for example, by nucleic acid homopolymers of poly-adenine (An), poly-thymine (Tₙ), polycytosine (Cₙ), poly-guanine (Gₙ), and poly-uridine (Uₙ). Repeating sequences also can include mixed polymers, such as AT repeats ([AT]ₙ), and the like.

The number of bases of the repeating base sequence of the one or more heterologous nucleotides of the isolated nucleic acid molecule can be equal to, greater than, or less than the number of bases of the repeating base sequence of the purification probe. The lengths of the complementary repeating sequences can determine the melting temperature (Tₘ) of the heterologous segment:purification probe complex. In one embodiment, the repeating base sequence of the heterologous segment is longer than the complementary repeating base sequence of the purification probe. In another embodiment, the repeating base sequence of the heterologous segment or the purification probe can be at least about 5 bases in length, illustratively about 5 to about 40, about 10 to about 30, or about 15 to about 20, and the like.

In other embodiments, the one or more heterologous nucleotides comprise an operably linked control sequence. In one embodiment, the control sequence is an enhancer or a promoter sequence that is specifically recognized by an RNA polymerase that binds to that sequence and initiates transcription to produce RNA transcripts. Non-limiting examples of promoters recognized by an RNA polymerase include promoters such as T3, T7, or SP6. Thus, an isolated nucleic acid molecule can be used in a variety of nucleic acid based assays including assays that use an RNA polymerase to produce multiple RNA transcripts such as, for example, transcription-mediated amplification (TMA) assay as described in Nature 350:91-92 (1991); and U.S. Patent No. 5,399,491.

In one embodiment, the isolated nucleic acid sequences of the present invention are labeled, e.g. labeled radioactively, chemiluminescently, fluorescently, phosphorescently or with infrared dyes or with a surface-enhanced Raman label or plasmon resonant particle (PRP). For example, modifications of nucleotides include the addition of acridine or derivatives thereof, Acrydite™, amine, biotin, BHQ-1™, BHQ-2™, BHQ-3™, borane dNTPs, carbon spacers (*e.g.,* C₃, C₆, C₇, C₉, C₁₂ or C₁₈), cascade blue, cholesterol, coumarin or derivatives thereof, Cy3®, Cy3.5®, Cy5®, Cy5.5®, Cy7® DABCYL, dansylchloride, digoxigenin, dinitrophenyl, dual biotin, EDANS, 6-FAM, fluorescein, 3'-glyceryl, HEX, IAEDANS, inverted dA, inverted dG, inverted dC, inverted dG, IRD-700, IRD-800, JOE, La Jolla Blue, metal clusters such as gold nanoparticles, phenylboronic acid, phosphate psoralen, 3'- or 5'-phosphorylation, pyrene, 3' ribo-adenosine, 3' ribo-guanosine, 3' ribocytidine, (LC)Red640, (LC)Red705, rhodamine, ROX, thiol (SH), spacers, TAMRA, TET, AMCA-S®, SE, BODIPY®, Marina Blue®, Oregon Green®, Pacific Blue®, QSY7™, Rhodamine Green®, Rhodamine Red®, Rhodol Green®, tetramethylrhodamine, Texas Red®, Uni-Link NH₂-modifier, radiolabels (*e.g*., ¹²⁵I, ¹³¹I, ³⁵S, ¹⁴C, ³²P, ³³P, ³H) and nanoparticles. A variety of labeling techniques are known to one of ordinary skill in the art.

Labels can be joined directly or indirectly to the isolated nucleic acid molecule. The labeling of a nucleic acid can be performed by covalently attaching a detectable group (label) to either an internal or terminal position, for example. One skilled in the art knows that there are a variety of ways for derivatizing oligonucleotides with reactive functionalities that permit the addition of a label. A number of approaches are available for directly attaching labels to nucleic acid molecules and for biotinylating probes so that radioactive, fluorescent, chemiluminescent, enzymatic, or electron dense labels can be attached via avidin. Non-limiting examples of references describing labels and methods for labeling nucleic acids include U.S. Patent No. 4,605,735; U.S. Patent No. 4,757,141; U.S. Patent No. 6,965,020; Nucl. Acids Res. 5:363 (1978); Nucl. Acids Res. 13:1529 (1985); Nucl. Acids Res. 15:3131 (1987); Nucl. Acids Res. 15:6455 (1987); Nucl. Acids Res. 13:4485 (1985); Nucl. Acids Res. 15:4837 (1987); and Anal. Biochem. 169:1-25 (1988).

In some embodiments, the isolated nucleic acid molecules are labeled for detecting methods using fluorescence resonance energy transfer (FRET). FRET involves two dyes, a donor and acceptor dye. FRET can be detected by either fluorescence of the acceptor dye ("sensitized fluorescence") if said acceptor is itself fluorescent, or by quenching of the donor dye fluorescence if said acceptor is a quenching non-fluorescent dye. FRET can be delayed if the donor dye releases its fluorescence over time. This process is termed "TR-FRET" or "time-resolved FRET". Donor and acceptor dyes can also be the same in which case FRET is detected by the resulting fluorescence depolarization. Dyes can also be covalently coupled to form a tandem fluorescent dye or tandem dye or tandem conjugate. For example, a single donor dye is then capable of exciting two acceptor dyes simultaneously, leading to the emission of light of multiple wavelengths. Preferably, the donor emission wavelength profile should at least partially overlap with the acceptor absorption wavelength profile.

Fluorescent dyes that can be employed include, but are not limited, BODIPY FL, Cy3®, Cy3.5®,Cy5.RTM., Cy5.5®, EDANS, FAM, fluorescein, HEX, IAEDANS, JOE, Oregon Green®, (LC)Red640, (LC)Red705, ROX, TAMRA, TET, tetramethylrhodamine and Texas Red®.

Quencher dyes include, but are not limited to, BHQ-1™, BHQ-2™, BHQ-3™, DABCYL, metal clusters such as gold nanoparticles and QSY7™.

Donor/acceptor pairs that can be employed include, but are not limited to, FAM/BHQ-1, TET/BHQ-1, JOE/BHQ-1, HEX/BHQ-1, Oregon Green/BHQ-1, TAMRA/BHQ-2, ROX/BHQ-2, Cy3/BHQ-2, Cy3.5/BHQ-2, Texas Red/BHQ-2, Texas Red/BHQ-2, Cy5/BHQ-3, Cy5.5/BHQ-3 fluorescein/tetramethylrhodamine, fluorescein/fluorescein, fluorescein/QSY7, fluorescein/LC RED640, fluorescein/LC Red705 IAEDANS/fluorescein, EDANS/DABCYL, and BODIPY FLI/BODIPY FL.

Also described is an isolated nucleic acid molecule consisting of a nucleotide sequence as set forth in SEQ ID NO: 1, SEQ ID NO:2, or SEQ ID NO:3, or a complement thereof, wherein the nucleic acid molecule further comprises a detectable label.

In some embodiments, the detectable label corresponds to a donor/acceptor pair suitable for detecting using FRET.

In other embodiments, the donor/acceptor pair is FAM/BHQ-1.

In still further embodiments, the present invention provides an isolated nucleic acid molecule comprising a nucleotide sequence as set forth in SEQ ID NO:3, wherein the nucleic acid molecule comprises FAM at the 5' end and BHQ-1 at the 3' end.

In other embodiments, the nucleic acid molecules of the present invention can provide for simultaneous use of two or more probes using donor-acceptor energy transfer whereby, e.g., molecular beacons are prepared that possess differently colored fluorophores, enabling assays to be carried out that simultaneously detect different targets in the same reaction. For example, multiplex assays can contain a number of different primer sets, each set enabling the amplification of a unique gene sequence from a different HAV, and a corresponding number of molecular beacons can be present, each containing a probe sequence specific for one of the amplicons, and each labeled with a fluorophore of a different color. The color of the resulting fluorescence, if any, identifies the HAV in the sample, and the number of amplification cycles required to generate detectable fluorescence provides a quantitative measure of the number of target organisms present. If more than one type of HAV is present in the sample, the fluorescent colors that occur identify which are present.

In other aspects, the present invention provides a composition comprising one or more of the isolated nucleic acid molecules of the present invention. In some embodiments, the composition is a buffered solution. In other embodiments, the composition is lyophilized.

In one embodiment, the composition comprises a pair of primers having the sequence as set forth in (SEQ ID NO:1 / SEQ ID NO:2).

In one embodiments, the composition further comprises a probe having the sequence as set forth in SEQ ID NO:3, or a complement thereof.

In another embodiment, a composition is provided comprising a first, a second, and/or a third pair of primers, wherein the first pair of primers has the sequence as set forth in (SEQ ID NO:1 SEQ ID NO:2). In one embodiment, the composition further comprises of a first, a second, and/or a third probe, wherein the first, the second and/or the third probe each comprise a detectable label suitable for use in a multiplex real time PCR.

In other embodiments, the composition comprises a pair of primers having the sequence as set forth in (SEQ ID NO:1 SEQ ID NO:2) and a probe having the sequence as set forth in SEQ ID NO:3, or a complement thereof.

In one embodiment, the composition comprises, in addition to the one or more nucleic acid molecules of the present invention, additional reagents such as DNA polymerase, cofactors, and deoxyribonucleoside-5'-triphosphates in suitable concentrations to provide amplification of the target nucleic acid. By way of example, in some embodiments, wherein the composition is a PCR solution, the minimal amount of DNA polymerase can be at least about 0.5 units/100 µl of solution, illustratively, about 0.5 to about 25 units/100 µl of solution and about 7 to about 20 units/100 µl of solution. Other amounts may be useful for a given amplification reaction or system. The "unit" can be defined as the amount of enzyme activity required to incorporate 10 nmoles of total nucleotides (dNTP's) into an extending nucleic acid chain in 30 minutes at 74 °C. By way of another example, in other embodiments, the amount of each primer used in amplification can be at least about 0.075 µmolar, illustratively, about 0.075 to about 2 µmolar, but other amounts may be useful for a given amplification reaction or system. By way of a still further example, in some embodiments, the amount of each dNTP in the solution can be about 0.25 to about 3.5 mmolar, but other amounts may be useful for a given amplification reaction or system.

In other aspects, the present invention provides a method for amplifying a target sequence corresponding to an HAV, as defined in claim 4.

. For example, performing a PCR with the target sequence and at least a forward primer and a reverse primer each capable of annealing to the target sequence under a suitable PCR condition can provide for amplification of the target sequence.

In one embodiment, the present invention provides a method for amplifying a target sequence, the method comprising: performing a PCR with the target sequence as template, wherein performing comprises providing the PCR with a forward primer comprising the sequence as set forth in SEQ ID NO:1 and a reverse primer comprising the sequence as set forth in SEQ ID NO:2. In one embodiment, the target sequence corresponds to cDNA, prepared from RNA of a sample comprising an HAV.

In other aspects, the present invention provides a method for determining HAV in a, as defined in claim 6. For example, the sample may comprise HAV RNA or the sample may be a sample in which the presence or absence of the HAV is to be deteremined.

In one embodiment, the present invention provides a method for determining HAV in a sample, the method comprising:
a. performing a PCR with a nucleic acid template in the sample using a forward primer comprising the sequence as set forth in SEQ ID NO:1 and a reverse primer comprising the sequence as set forth in SEQ ID NO:2; and
b. detecting an amplicon generated by the forward and the reverse primer, wherein the presence of the amplicon determines the HAV in the sample.

In one embodiment, the template is a cDNA prepared from RNA of a sample comprising an HAV.

The step of detecting can be performed by a number of techniques known to one of ordinary skill in the art. In one embodiment, the amplicon can be detected using a probe that is labeled for detection and can be directly or indirectly hybridized with the amplicon. The probe may be soluble or attached to a solid support.

In one embodiment, the probe comprises a detectable label corresponding to a donor/acceptor pair suitable for detecting using FRET, wherein the probe comprises a sequence as set forth in SEQ ID NO:3, or complements thereof. For example, in some embodiments, the donor/acceptor pair is FAM/BHQ-1.

In another embodiment, one or more of the primers used to amplify the target nucleic acid can be labeled, for example, with a specific binding moiety. The resulting primer extension product into which the labeled primer has been incorporated can be captured with a probe. Detection of the amplified target hybridized to the probe can be achieved by detecting the presence of the labeled probe or labeled amplified target using suitable detection equipment and procedures that are well known in the art.

In other embodiments, one or more of the primers used to amplify the target nucleic acid is labeled with biotin and the biotinylated amplified target nucleic acids are hybridized to probes attached to a solid support. The bound targets are then detected by contacting them with a streptayidin-peroxidase conjugate in the presence of an oxidant, such as hydrogen peroxide, and a suitable dye-forming composition.

Other techniques are known to one of ordinary skill in the art for detecting including, but not limited to, methods involving southern blotting, dot blot techniques, or nonisotopic capture detection with a labeled probe.

In other embodiments, the present invention provides a method for determining HAV in a sample, the method comprising:
a. performing a single PCR with the sample, the PCR comprising a forward primer comprising the sequence as set forth in SEQ ID NO:1 and a reverse primer comprising the sequence as set forth in SEQ ID NO:2; and
b. detecting an amplicon generated by the forward and the reverse primers, wherein the presence of the amplicon determines the HAV in the sample.

In one embodiment, the step of detecting comprises including in the PCR an oligonucleotide probe comprising a detectable label corresponding to a donor/acceptor pair suitable for detecting using FRET, wherein the probe comprises a sequence as set forth in SEQ ID NO:3, or a complement thereof. For example, in some embodiments, the donor/acceptor pair is FAM/BHQ-1.

Thus, the nucleic acid molecules of the present invention can be employed singly or in combination in a variety of methods for amplifying and/or determining HAV. Accordingly, in some embodiments, the compositions, methods, and kits of the present invention provide for a PCR assay, a realtime PCR assay, and/or a multiplex real time PCR assay, wherein depending on the type of assay, the assay may comprise one, two, three or more sets of primers and probes in the same PCR master mix. For example, in some embodiments, a multiplex real time PCR assay can detect different HAV genotypes using a single test. In this regard, the primers and probes are capable of interacting only with their specific target and not with other primers and probes present in the master mix *(e.g.,* do not form primer-dimers) thereby providing for efficient target amplification and detection in PCR, e.g., multiplex PCR.

In other aspects, the primers/probes of the present invention may be used in quantitative methods. Quantification of HAV may be, for example, by semi-quantitative or quantitative methods, such as those known to one of ordinary skill in the art.

In one embodiment, semi-quantitative RT-PCR results may be generated by sampling of the RT-PCR reaction mixture followed by, *e.g.,* dot-blot analysis.

In another embodiment, quantitative procedures may use non-competitive or competitive RT-PCR techniques.

Noncompetitive RT-PCR may include, for example, the co-amplification of the target HAV RNA with a second RNA molecule under reagent concentrations and conditions so that there is no competition between target and standard, and with which it shares neither the primer recognition sites nor any internal sequence.

In competitive RT-PCR, the internal standard shares the same primer recognition and internal sequences with the primary target, leading to competition for reagents. Both can be amplified with the same or substantially the same efficiency and their amplicons can be distinguished by the addition of a restriction enzyme site to the standard, by varying its size, etc. For example, in some embodiments, a series of PCR tubes containing the target HAV RNA are spiked with serial dilutions of known copy numbers of the internal standard. The greater the concentration of the internal standard, the more likely it is that the primers will bind and amplify it, rather than the target. A comparison of the intensities of ethidium-bromide-stained standard and target amplicons, for example following gel electrophoresis, allows target quantification.

In one embodiment, the method may involve spiking into the HAV RNA samples before RT of known amounts of RT-PCR-amplifiable competitors that, preferably, are synthetic HAV RNA molecules.

In another embodiment, the present invention provides a quantitative real time PCR assay, *e.g.,* an end point PCR assay followed by detection with the probe sequence described herein using southern blot, dot blot, fluorescent, or colorimetric detection or other known qualitative or quantitative methods for nucleic acid detection.

In other aspects, the present invention provides a kit comprising the isolated nucleic acid molecules including the primers and probes of the present invention. The kit can be developed using the nucleic acid sequences disclosed herein. These sequences can be used as primers in nucleic acid amplification reactions, and/or as probes in a nucleic acid hybridization method. The kits are useful for determining the presence of a HAV nucleic acid in a sample. Components in the kit can either be obtained commercially or made according to well known methods in the art. In addition, the components of the kit can be in solution or lyophilized as appropriate. In one embodiment, the components are in the same compartment, and in another embodiment, the components are in separate compartments. In some embodiments, the kit further comprises instructions for use.

In one embodiment, the kit comprises a forward primer, a reverse primer, and a probe, wherein the forward primer comprises a forward primer nucleic acid sequence as set forth in (SEQ ID NO: 1), wherein the reverse primer comprises a reverse primer nucleic acid sequence as set forth in (SEQ ID NO: 2), wherein the probe comprises a probe nucleic acid sequence as set forth in (SEQ ID NO:3), or a complement thereof.

The following examples are provided for illustration only.

### EXAMPLES

### Example 1

### Determining HAV by RT-PCR

To determine the presence of HAV RNA in a plasma sample, a RT-PCR assay was performed.

The primers and detection probe having the following sequences were employed:
Forward primer: 5'-GCG CCC GGC GGG GTC AAC TCC AT-3' (SEQ ID NO:1);
Reverse primer: 5'-AGC CAA GTT AAC ACT GCA AGG-3' (SEQ ID NO:2);
Probe: 5'-TTA GCA TGG AGC TGT AGG AGT CTA AAT TGG GG -3' (SEQ ID NO:3).

The probe was labeled with FAM at the 5' end and BHQ-1 at the 3' end.

An RT-PCR master mix (MMX) was prepared comprising the following: Invitrogen 2x PCR reaction mix; 400 µM dNTPs; 4.0 mM Macl₂; 1x ROX reference dye (0.5 µM); 400 nM forward primer; 400 nM reverse primer; 50 nM probe; 100 nM Internal Control probe; 1 µL/50 µL PCR reaction of Invitrogen SSIII One Step Reverse Transcriptase (RT) and *Taq* DNA polymerase blend (Invitrogen, Carlsbad, CA).

The MMX was combined with viral RNA isolated from plasma samples containing the virus using a virus extraction method known in the art. The combined HAV RNA / MMX was subjected to one step PCR, where the reverse transcription, amplification of cDNA, and detection occured in the same tube, using a commercial real time PCR instrument (Applied Biosystems 7300). The thermal cycling conditions are shown in Table 1:

**Table 1: PCR cycling condition.**

| **Temperature** | **Time** | **Cycles** |
|---|---|---|
| 55°C | 30 min | 1 |
| 95°C | 3 min | 1 |
| 95°C | 15 sec | 45 |
| 56°C | 1 min | |

Following PCR amplification the signals generated were analyzed using the instrument software. The results showed strong amplification curves.

## Claims

1. An isolated nucleic acid molecule consisting of a nucleotide sequence, or a complement thereof, as set forth in:
5'-GCG CCC GGC GGG GTC AAC TCC AT-3' (SEQ ID NO:1);
5'-AGC CAA GTT AAC ACT GCA AGG-3' (SEQ ID NO:2); or
5'- TTA GCA TGG AGC TGT AGG AGT CTA AAT TGG GG-3'(SEQ ID NO:3).

2. A composition comprising a pair of oligonucleotide primers comprising a forward primer having a sequence as set forth in SEQ ID NO:1 and a reverse primer having a sequence as set forth in SEQ ID NO:2, wherein the pair of primers are capable of annealing to a target sequence under a PCR condition to amplify the target sequence.

3. The composition of claim 2 further comprising an oligonucleotide probe having the sequence as set forth in SEQ ID NO:3, wherein the probe is capable of annealing to the target sequence.

4. A method for amplifying a target sequence, the method comprising:
performing a PCR with the target sequence as template, wherein performing comprises providing the PCR with a forward primer comprising the sequence as set forth in SEQ ID NO:1 and a reverse primer comprising the sequence as set forth in SEQ ID NO:2.

5. The method of claim 4, wherein the target sequence corresponds to a cDNA prepared from RNA of a sample comprising an HAV.

6. A method for determining HAV in a sample, the method comprising:
a. performing a PCR with a nucleic acid template in the sample using a forward primer comprising the sequence as set forth in SEQ ID NO:1 and a reverse primer comprising the sequence as set forth in SEQ ID NO:2; and
b. detecting an amplicon generated by the forward and the reverse primer, wherein the presence of the amplicon determines whether the HAV is present in the sample.

7. The method of claim 6, wherein detecting comprises providing a polynucleotide probe to the PCR whereby the amplicon, if present, contacts the probe, wherein the probe comprises the sequence as set forth in SEQ ID NO:3 or a complement thereof.

8. The method of claim 7, wherein the probe is labeled with FAM/BHQ-1.

9. A kit comprising one or more of the isolated nucleic acid molecules of claim 1.

## Patentansprüche

1. Isoliertes Nukleinsäuremolekül, bestehend aus einer Nukleotidsequenz, oder einer dazu komplementären Sequenz, wie nachstehend definiert:
5'-GCG CCC GGC GGG GTC AAC TCC AT-3' (SEQ. ID. NR. 1);
5'-AGC CAA GTT AAC ACT GCA AGG-3' (SEQ. ID. NR. 2); oder
5'-TTA GCA TGG AGC TGT AGG AGT CTA AAT TGG GG-3' (SEQ. ID. NR. 3).

2. Zusammensetzung umfassend ein Paar Oligonukleotid-Primer bestehend aus einem Vorwärtsprimer mit einer Sequenz gemäß SEQ. ID. NR. 1 und einem Rückwärtsprimer mit einer Sequenz gemäß SEQ. ID. NR. 2, wobei das Primerpaar in der Annealingphase einer Polymerase-Kettenreaktion (PCR) zur Anlagerung an eine Zielsequenz fähig ist, um diese Zielsequenz zu amplifizieren.

3. Zusammensetzung nach Anspruch 2, weiterhin umfassend eine Oligonukleotid-Sonde mit der in SEQ. ID. NR. 3 angegebenen Sequenz, wobei die Sonde fähig ist, sich in einer Annealingphase an die Zielsequenz anzulagern.

4. Verfahren zur Amplifikation einer Zielsequenz, mit folgenden Schritten:
Durchführen einer PCR mit der Zielsequenz als Templat, wobei zur Durchführung der PCR ein Vorwärtsprimer mit der in SEQ. ID. NR. 1 angegebenen Sequenz und ein Rückwärtsprimer mit der in SEQ. ID. NR. 2 angegebenen Sequenz bereitgestellt wird.

5. Verfahren nach Anspruch 4, bei der die Zielsequenz einer cDNA entspricht, die aus der RNA einer Probe hergestellt wurde, die Hepatitis-A-Virus (HAV) enthielt.

6. Verfahren zum Nachweis von HAV in einer Probe, mit folgenden Schritten:
a. Durchführen einer PCR mit einem Nukleinsäure-Templat in der Probe unter Verwendung eines Vorwärtsprimers mit der in SEQ. ID. NR. 1 angegebenen Sequenz und eines Rückwärtsprimers mit der in SEQ. ID. NR. 2 angegebenen Sequenz;und
b. Detektion eines von den Vorwärts- und Rückwärtsprimern erzeugten Amplikons, wobei durch das Vorhandensein des Amplikons nachgewiesen wird, ob HAV in der Probe enthalten ist.

7. Verfahren nach Anspruch 6, bei dem der HAV-Nachweis das Bereitstellen einer Polynukleotid-Sonde für die PCR umfasst, wodurch das Amplikon, soweit vorhanden, die Sonde kontaktiert, wobei die Sonde die in SEQ. ID. NR. 3 angegebene Sequenz oder eine dazu komplementäre Sequenz hat.

8. Verfahren nach Anspruch 7, bei dem die Sonde mit FAM/BHQ-1 markiert ist.

9. Kit mit einem oder mehreren der isolierten Nukleinsäuremoleküle gemäß Anspruch 1.

## Revendications

1. Molécule d'acide nucléique isolée consistant en une séquence nucléotidique, ou un complément de celle-ci, telle que représentée dans :
5'-GCG CCC GGC GGG GTC AAC TCC AT-3' (SEQ ID NO:1);
5'-AGC CAA GTT AAC ACT GCA AGG-3' (SEQ ID NO:2); ou
5'-TTA GCA TGG AGC TGT AGG AGT CTA AAT TGG GG-3' (SEQ ID NO:3).

2. Composition comprenant une paire d'amorces oligonucléotidiques comprenant une amorce sens ayant une séquence telle que représentée dans SEQ ID NO:1 et une amorce anti-sens ayant une séquence telle que représentée dans SEQ ID NO:2, dans laquelle la paire d'amorces est capable de se combiner à une séquence cible sous une condition de PCR pour amplifier la séquence cible.

3. Composition de la revendication 2 comprenant en outre une sonde oligonucléotidique ayant la séquence telle que représentée dans SEQ ID NO:3, dans laquelle la sonde est capable de s'hybrider à la séquence cible.

4. Procédé pour amplifier une séquence cible, le procédé comprenant :
la réalisation d'une PCR avec la séquence cible comme modèle, dans laquelle la réalisation comprend de fournir à la PCR une amorce sens comprenant la séquence telle que représentée dans SEQ ID NO:1 et une amorce anti-sens comprenant la séquence telle que représentée dans SEQ ID NO:2.

5. Procédé de la revendication 4, dans lequel la séquence cible correspond à un cDNA préparé à partir d'un RNA d'un échantillon comprenant un HAV.

6. Procédé pour détecter le HAV dans un échantillon, le procédé comprenant :
a. la réalisation d'une PCR avec un acide nucléique modèle dans l'échantillon en utilisant une amorce sens comprenant la séquence telle que définie dans SEQ ID NO:1 et une amorce anti-sens comprenant la séquence telle que définie dans SEQ ID NO:2; et
b. la détection d'un amplicon généré par l'amorce sens et l'amorce anti-sens, dans laquelle la présence de l'amplicon détermine si le HAV est présent dans l'échantillon.

7. Procédé de la revendication 6, dans lequel la détection comprend la fourniture d'une sonde polynucléotidique à une PCR par laquelle l'amplicon, s'il est présent, entre en contact avec la sonde, dans laquelle la sonde comprend la séquence telle que définie dans SEQ ID NO:3 ou un complément de celle-ci.

8. Procédé de la revendication 7, dans lequel la sonde est marquée avec FAM/BHQ-1.

9. Trousse comprenant l'une au moins des molécules d'acide nucléique isolées de la revendication 1.
